(19) Europäisches Patentamt

European Patent Office

Office européen des brevets

(11) Veröffentlichungsnummer: **0 387 850 B1**

(12) **EUROPÄISCHE PATENTSCHRIFT**

(45) Veröffentlichungstag der Patentschrift: **31.05.95**

(21) Anmeldenummer: **90104839.7**

(22) Anmeldetag: **14.03.90**

(51) Int. Cl.⁶: **C12P 21/08**, C12N 5/24, A61K 39/40, G01N 33/577

(54) **Monoklonale, gegen ECA gerichtete Antikörper und ihre Verwendung.**

(30) Priorität: **15.03.89 DE 3908520**
**28.07.89 DE 3925161**

(43) Veröffentlichungstag der Anmeldung:
**19.09.90 Patentblatt 90/38**

(45) Bekanntmachung des Hinweises auf die
Patenterteilung:
**31.05.95 Patentblatt 95/22**

(84) Benannte Vertragsstaaten:
**AT BE CH DE DK ES FR GB GR IT LI LU NL SE**

(56) Entgegenhaltungen:
**GB-A- 2 186 592**

**JOURNAL OF CLINICAL MICROBIOLOGY, Band 25, Nr. 2, Februar 1987, American Society for Microbiology, Washington, DC (US); E.C. BÖTTGER et al., Seiten 377-382&NUM;**

**INFECTION & IMMUNITY, Band 50, Nr. 2, November 1985, American Society for Microbiology, Washington, DC (US); H. PETERS et al., Seiten 459-466&NUM;**

(73) Patentinhaber: **BOEHRINGER MANNHEIM GMBH**

**D-68298 Mannheim (DE)**

(72) Erfinder: **Brandt, Michael, Dr. rer. nat.**
**Hofmairstrasse 19**
**D-8132 Tutzing (DE)**
Erfinder: **Endl, Josef, Dr. rer. nat.**
**Ulmenstrasse 12**
**D-8120 Weilheim (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**Beschreibung**

Die Erfindung betrifft gegen ECA gerichtete spezifische Antikörper, die z.B. aus den Zellinien ECACC 89030310 und ECACC 89030311 gewonnen werden, sowie deren Verwendung.

In der Familie der Enterobacteriaceae, die gram-negative, begeißelte Stäbchen, die fakultativ aerob sind, umfaßt, sind einige pathogene Mikroorganismen wie Escherichia, Erwinia, Serratia, Proteus, Salmonella und Shigella zusammengefaßt. Die von diesen Mikroorganismen verursachten Infektionen vermehren sich insbesondere im klinischen Bereich sehr stark und darüberhinaus entwickeln diese Stämme eine Resistenz gegen Antibiotika, so daß Infektionen sehr schwer zu bekämpfen sind. Die Enterobacteriaceae-Infektionen betreffen einerseits die aufsteigenden Harnwege und andererseits das gastroenteritische System. Insbesondere durch Salmonella typhimurium, dem für eine Nahrungsmittelvergiftung typischen Keim, werden viele Todesfälle hervorgerufen. Man versucht daher, ein gegen diese Mikroorganismen wirksames Antiserum zu finden.

Ein Ansatzpunkt dazu bot sich in dem allen Enterobacteriaceaen gemeinsamen, als Enterobacterial Common Antigen (ECA) bezeichneten Antigen. Es wurde versucht entweder mit dem kompletten Mikroorganismus oder mit diesem Antigen Tiere zu immunisieren und anschließend entweder direkt polyklonale Antikörper oder nach Fusion monoklonale Antikörper zu gewinnen. Die so erhaltenen Antikörper zeigten jedoch keinen Schutz gegen Enterobacteriaceae-Infektionen. So ist in FEMS Microbiology Letters 27 (1985), 307-312 angegeben, daß ein aus Kaninchen erhaltenes Anti-ECA-Serum bei Mäusen keinen Schutz gegen Infektionen bot. Weiterhin wird in Infection and Immunity, 2 (1970) 175-182 die Herstellung von Antikörpern beschrieben, die jedoch nur mit dem Common Antigen von ganz bestimmten einzelnen Mikroorganismen reagieren.

Gegen ECA gerichtete monoklonale Antikörper sind auch aus J Clin Microbiol 25 (1987) 377-382 und Infection and Immunity 50 (1985) 459-466 bekannt. Die Herstellung von gegen O-Seitenketten auf den LPS-Molekülen gerichteten monoklonalen Antikörper, die mit mehreren Enterobakterien-Gattungen kreuzreagieren, wird in GB-A-2185592 beschrieben. Die monoklonalen Antikörper werden durch die Verwendung von aus dem Blut von infizierten Spendern genomen Lymphozyten produziert.

Es war daher Aufgabe der Erfindung, Antikörper, die gegen Enterobacteriaceae und insbesondere gegen die pathogenen Gattungen dieser Familie gerichtet sind, zur Verfügung zu stellen. Weiterhin war es Aufgabe der Erfindung, eine Möglichkeit zur Behandlung und zum Nachweis von Enterobacteriaceae-Infektionen zu schaffen.

Diese Aufgabe wird gelöst durch ein Verfahren zur Herstellung von gegen ECA gerichteten monoklonalen Antikörpern, das dadurch gekennzeichnet ist, daß man aus dem Blut von Spendern, bei denen eine Enterobacteriaceae-Infektion nachgewiesen wurde, Lymphozyten gewinnt, die Lymphozyten immortalisiert, die Zellen, die Antikörper mit der gewünschten Aktivität produzieren, selektiert, züchtet und die Antikörper daraus gewinnt.

Überraschenderweise gelingt es durch Verwendung von geeigneten humanen Lymphozyten Antikörper zu produzieren, die spezifisch an Enterobacteriaceae binden können.

Die erfindungsgemäßen Zellinien, die durch Fusion von humanen B-Lymphozyten mit Myelomzellen erhalten wurden, zeigen nicht nur eine spezifische Bindung mit ECA, sondern binden überraschenderweise auch an Mikroorganismen der Familie Enterobacteriaceae.

Zur Herstellung von gegen Enterobacteriaceae gerichteten Antikörpern werden aus dem Blut erkrankter Personen, bei denen eine Enterobacteriaceae-Infektion nachgewiesen wurde, nach bekannten Methoden B-Lymphozyten gewonnen. Die Lymphozyten werden dann immortalisiert. Zur Immortalisierung stehen verschiedene Methoden zur Verfügung. Die humanen Lymphozyten können mit Myelomzellen humaner oder muriner Herkunft nach der Methode von Köhler und Milstein fusioniert werden. Zur Fusion können aber auch Heteromyelome verwendet werden. Ebenso ist es möglich, die B-Lymphozyten mit Epstein Barr Virus zu transformieren. Weiterhin kann das in DE-A 32 45 665 oder das in EP-A-0 256 512 beschriebene Verfahren angewendet werden. Dabei wird mit subzellulären Vesikeln, die eine transformierende DNA enthalten, fusioniert. Bevorzugt werden die B-Lymphozyten durch Fusion mit Zellen der humanen Myelomzellinie SKO-007, ATCC CRL 8033 immortalisiert.

Von den immortalisierten Zellen werden diejenigen ausgewählt, die Antikörper mit der gewünschten Aktivität produzieren. Die Selektionsmethoden sind dem Fachmann hinreichend bekannt und bedürfen hier keiner näheren Erläuterung. Geeignet ist beispielsweise der Nachweis der Bindung mit Lipopolysaccharid (LPS)-Präparationen der Enterobacteriaceaen über einen ELISA-Test, da die meisten kommerziell erhältlichen LPS-Präparate auch substantielle Mengen an ECA enthalten.

Die Zellen, die die gewünschten Antikörper produzieren, werden dann gezüchtet und die Antikörper in an sich bekannter Weise gewonnen.

Gegenstand der Erfindung sind auch die unter den Nummern ECACC 89030310 (L30) und 89030311 (E21) hinterlegten Zellinien, die monoklonale Antikörper, die spezifisch mit ECA reagieren, produzieren.

Ein weiterer Gegenstand der Erfindung ist die Verwendung der aus den beanspruchten Zellinien gewonnenen monoklonalen Antikörper zur Herstellung eines pharmazeutischen Präparats zur passiven Immunisierung gegen von Enterobacteriaceae hervorgerufenen Infektionen.

Da die nach dem erfindungsgemäßen Verfahren erhaltenen monoklonalen Antikörper spezifisch an lebende Enterobacteriaceae binden können, können sie zur Bekämpfung dieser Mikroorganismen im menschlichen Organismus eingesetzt werden. Dabei ist es sowohl möglich, Patienten zur Vorbeugung die monoklonalen Antikörper zu verabreichen als auch nach einer erfolgten Infektion die gewonnenen monoklonalen Antikörper zur Behandlung einzusetzen. Eine zur passiven Immunisierung geeignete Dosis liegt im Bereich von 50 bis 200 mg.

Da die nach dem erfindungsgemäßen Verfahren gewonnenen monoklonalen Antikörper im wesentlichen mit den Gattungen der Familie Enterobacteriaceae bindefähig sind, eignen sich die Antikörper auch zum qualitativen oder quantitativen Nachweis von Enterobacteriaceae. Der Nachweis erfolgt dabei in an sich bekannter Weise durch ein immunologisches Bestimmungsverfahren. Verfahren dieser Art sind dem Fachmann bekannt und brauchen hier nicht näher erläutert zu werden. Der erfindungsgemäß gewonnene monoklonale Antikörper kann dabei als markierter und/oder immobilisierter Rezeptor eingesetzt werden.

Gegenstand der Erfindung ist weiterhin ein Arzneimittel zur Behandlung von durch Enterobacteriaceae hervorgerufenen Infektionen, enthaltend gemäß dem erfindungsgemäßen Verfahren erhaltene, bevorzugt aus den Zellinien ECACC 89030310 und/oder 89030311 gewonnene monoklonale Antikörper zusammen mit üblichen Träger- und Verdünnungsmitteln.

Bevorzugt wird monoklonaler Antikörper in einer Dosis von 50 bis 200 mg eingesetzt.

Erfindungsgemäß wird ein Arzneimittel zur Verfügung gestellt, mit dem gegen Antibiotika resistente Enterobacteriaceae-Stämme bekämpft werden können.

Die Erfindung wird durch die folgenden Figuren und Beispiele erläutert:

Fig. 1      zeigt ein Diagramm, in dem die Ergebnisse gezeigt sind, die nach Inkubieren mit verschiedenen Verdünnungen der Antikörper E21 mit ECA und verschiedenen LPS-Proben, die an Mikrotiternäpfe adsorbiert waren, im ELISA erhalten wurden.

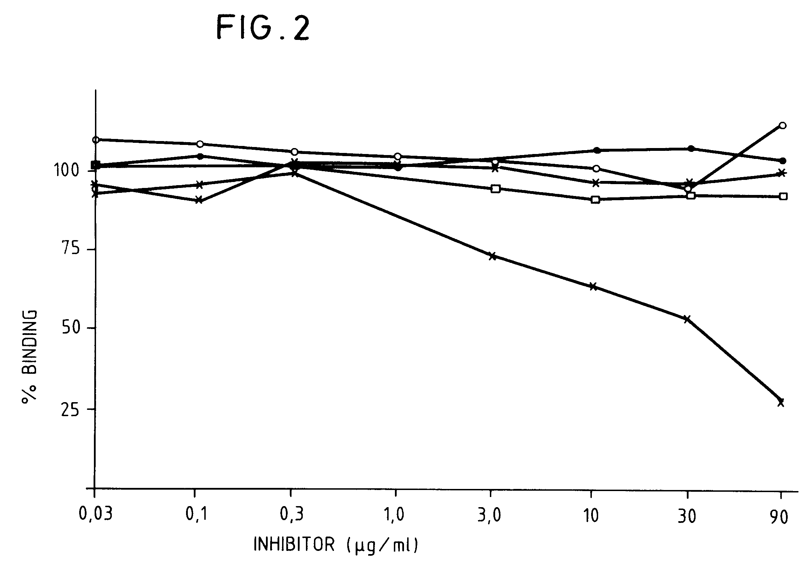

Fig. 2      zeigt ein Diagramm, in dem die Ergebnisse, die bei Inkubation von löslichem ECA oder löslichem LPS mit Mikrotiterplatten, die mit ECA aus S. montevideo beschichtet waren, erhalten wurden, aufgetragen sind. Antikörper E21 mit einer Konzentration von ca. 1 $\mu$g/ml wurde 1 Stunde bei 25°C inkubiert mit einem gleichen Volumen von löslichem ECA oder LPS. Die Mischung aus Antikörper und Inhibitor wurde auf die mit ECA aus S. montevideo beschichteten Mikrotiterplatten aufgebracht. Die Kurven zeigen die Ergebnisse, die im Vergleich zu der Bindung von Antikörpern an ECA erhalten wurden, wobei die Kurven für folgende LPS stehen:

x ECA (S. montevideo);

o LPS (S. montevideo);

* LPS (S. minnesota);

° LPS (E. coli 0111B4);

□ LPS (Serratia marcescens).

Fig. 3      zeigt ein Diagramm, in dem die Ergebnisse aufgetragen sind, die mit einem Versuch erhalten wurden, der wie bei Fig. 2 beschrieben durchgeführt wurde, wobei jedoch die Mikrotiterplatten mit LPS aus E. coli 0111B4 beschichtet waren.

Fig. 4      zeigt eine Immunoblot-Analyse. ECA und LPS wurden auf 14 % SDS/PAGE aufgetrennt. Das Gel im oberen Teil wurde mit Silber angefärbt, um das LPS-Wanderungsmuster zu zeigen. ECA wurde nicht angefärbt durch das Silberreagenz. Ein identisches Gel wurde verwendet für einen Immunoblot, der mit Antikörper E21 entwickelt wurde.

**Beispiel 1**

Für eine Auswahl der Spender, die mit hoher Wahrscheinlichkeit in vivo präaktivierte Lymphozyten gegen ECA aufweisen, wird folgendes Verfahren angewandt: Patienten mit einer Sepsis bzw. Bakteriämie wird Blut entnommen und nach bekannten bakteriologischen Verfahren eine Blutkultur angelegt. Aus dieser Blutkultur werden die Bakterien mit Hilfe eines Differenzierungsganges laut Bergey's Manual of Systematic Bacteriology (Bergey's Manual of Systematic Bacteriology (1984) N.R. Krieg, Cr. Holt, (eds.) Williams und Wilkins, Baltimore) identifiziert. Nur solche Personen, bei denen als Infektionsursache Bakterien aus der Familie der Enterobacteriaceae nachgewiesen wurden, kommen als Spender für Lymphozyten in Betracht.

Von diesen Spendern werden 100 bis 200 ml Blut abgenommen und daraus die mononuklearen Zellen isoliert. Anschließend werden diese Zellen nach herkömmlichen Methoden (Köhler und Milstein Nature 256 (1975) 495-497) mit einer humanen Myelom-Zellinie (SKO-007, ATCC CRL 8033) fusioniert. Nach 2 bis 3 Wochen werden die Kulturüberstände der Hybridzellen getestet. Dazu wurde ein ELISA- Test mit gereinigtem ECA, isoliert aus Salmonella montevideo, durchgeführt. Hierzu wurden in 96well ELISA-Platten (Greiner) 100 $\mu$l ECA-Lösung (10 $\mu$g/ml) in 10 mM Natriumphosphatpuffer, pH 7,35 pipettiert und eine Stunde bei Raumtemperatur inkubiert. Nach dem Waschen der Mikrotiterplatten mit PBS erfolgte die Blockierung unspezifischer Bindungsstellen mit Rinderserumalbumin. Danach wurden 100 $\mu$l Kulturüberstand in die einzelnen Näpfe pipettiert und für eine Stunde bei Raumtemperatur inkubiert. Nach dem Waschen wurden 100 $\mu$l Peroxidase markierter Schaf-anti-Human-leichte- Ketten-Antikörper zugegeben und wiederum für eine Stunde bei Raumtemperatur inkubiert. Nach erneutem Waschen wurde die Enzymreaktion mit Peroxidasesubstrat (ABTS, Boehringer Mannheim, Best.Nr. 102946) gestartet. Nach 20 bis 60 Minuten bei Raumtemperatur wurden die Extinktionen bei 405 nm in einem Photometer bestimmt. Die Klone, die die gewünschten Antikörper produzieren, werden weitergezüchtet.

**Beispiel 2**

Um die Spezifität der aus der Zellinie ECACC 89030311 gewonnenen humanen monoklonalen Antikörper im Kulturüberstand der Hybridzellen zu ermitteln, wurde ein ELISA-Test, wie im Beispiel 1 beschrieben, durchgeführt. Dabei wurden die folgenden Ergebnisse erhalten:

Tabelle 1

| | ECA S. montevideo | mE erhalten mit | |
|---|---|---|---|
| | | LPS S. montevideo | LPS S. enteritidis |
| Klon E21 | 1520 | 10 | 100 |

**Beispiel 3**

Aus den Zellinien ECACC 89030310 und ECACC 89030311 gewonnene monoklonale Antikörper wurden auf ihre Bindefähigkeit mit intakten Bakterienzellen getestet. Dazu wurden mit Phenol abgetötete Bakterien an 96er Mikrotiterplatten durch Inkubation über Nacht bei 37°C adsorbiert. Danach wurden die Platten zweimal mit PBS gewaschen und mit 1% Crotein C (Boehringer Mannheim) eine Stunde bei Raumtemperatur nachbeschichtet. Nach dem Abkippen der Blockierungslösung wurden 100 $\mu$l der die monoklonalen Antikörper enthaltenden Lösung zugegeben und für eine Stunde bei Raumtemperatur inkubiert. Nach dreimaligem Waschen mit PBS wurden 100 $\mu$l Peroxidase-markierter Schaf-anti-human leichte Ketten Antikörper zugegeben und wiederum für eine Stunde bei Raumtemperatur inkubiert. Nach erneutem Waschen wurde die Bindung des Peroxidase-Konjugates mit ABTS nachgewiesen und die Farbreaktion im Photometer quantifiziert. Das Ergebnis ist der folgenden Tabelle 2 zu entnehmen:

T a b e l l e   2

| Klon | S. typhi-murium | Klebsiella pneumoniae Serotyp | | | Serratia marcescens | Proteus mirabilis | E. coli Serotyp | | Pseudomonas aeruginosa Serotyp | | | Bacillus subtilis | Lacto-bacillus plantarum |
|---|---|---|---|---|---|---|---|---|---|---|---|---|---|
| | | 2 | 3 | 31 | | | 01K1 | 07K1 | 01 | 06 | 016 | | |
| E21 | 0,194 | 0,145 | 1,130 | 0,745 | 0,806 | 0,998 | 0,670 | 0,498 | 0 | 0 | 0 | 0 | 0 |
| L30 | 0,108 | 0,059 | 0,490 | 0,420 | 0,365 | 0,552 | 0,275 | 0,165 | 0 | 0 | 0 | 0 | 0 |

Die Werte bedeuten mE bei einer Meßwellenlänge von 405 nm.

Diese Ergebnisse zeigen, daß die erfindungsgemäßen monoklonalen Antikörper mit einer Vielzahl von Mitgliedern der Familie Enterobacteriaceae binden, nicht aber mit nicht zu dieser Familie gehörigen Stämmen wie z.B. Pseudomonas.

**Beispiel 4**

Es wurde die immuntherapeutische Effizienz der aus der Zellinie ECACC 89030311 gewonnenen monoklonalen Antikörper getestet. Dazu wurden 4 bis 7 Wochen alten NMRI-Mäusen verschiedene Mengen an gereinigtem, aus der Zellinie ECACC 89030311 gewonnenem monoklonalem Antikörper intravenös verabreicht und 15 Minuten später eine konstante Menge an lebenden Bakterien intraperitoneal injiziert. Nach 48 Stunden wurde die Überlebensrate der Mäuse festgestellt. Tabelle 3 zeigt das Ergebnis:

Tabelle 3

| Klon | μg H-MAK/Maus | Organismus und Keimzahl | Protektionsrate (%) |
|---|---|---|---|
| E21 | 57 | | 80 (4/5) |
| | 14 | $1,5 \times 10^6$ CFU | 66 (2/3) |
| | 3,5 | Serratia marcescens | 0 (0/8) |
| | 1 | | 0 (0/8) |
| CA13/11 | 63 | $4 \times 10^6$ CFU Serratia marcescens | 0 (0/8) |
| CA13/11: Dieser MAK wurde als Kontrolle eingesetzt. Er reagiert mit dem O-Antigen von S. minnesota. | | | |

**Beispiel 5**

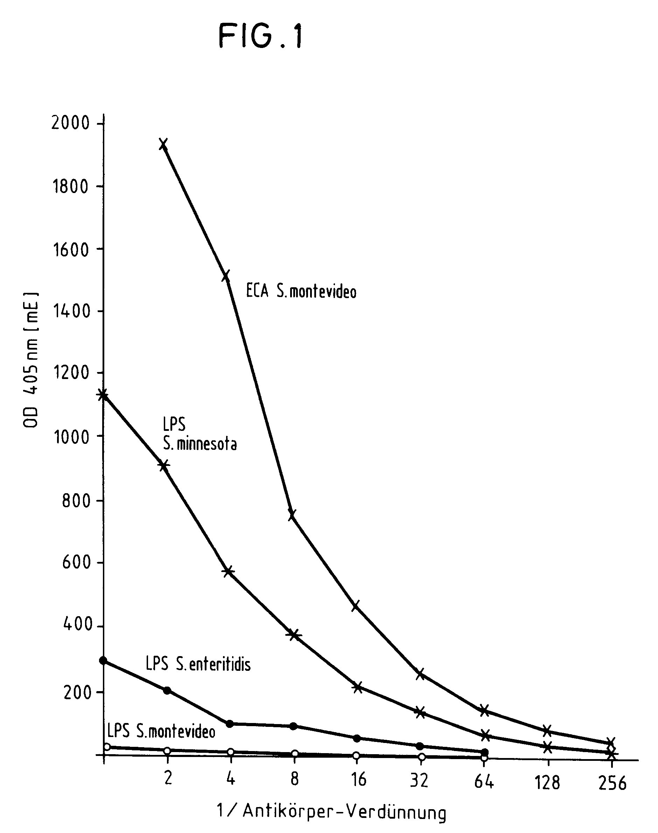

Es wurde untersucht, ob der aus der Zellinie ECACC 89030311 gewonnene Antikörper spezifisch mit ECA bindet, oder ob er auch eine Affinität zu Lipopolysacchariden an der äußeren Membran von Bakterien der Familie Enterobacteriaceae aufweist. Dazu wurde einerseits hochgereinigtes ECA eingesetzt und zum Vergleich die Bindung an Lipopolysaccharide, die aus verschiedenen Arten extrahiert worden waren, untersucht. ECA und Lipopolysaccharide (LPS) wurden in denselben Konzentrationen an Mikrotiterplatten adsorbiert und mit verschiedenen Verdünnungen des Antikörpers inkubiert. Anschließend wurde die Menge an gebundenem Antikörper über einen ELISA-Test bestimmt. Fig. 1 zeigt eine graphische Darstellung der Ergebnisse.

Bei jeder gegebenen Antikörperverdünnung wurde ein viel höheres ELISA-Signal mit immobilisiertem ECA als mit festphasengebundenem LPS erzeugt. Da diese Unterschiede bei der Reaktivität auch von der verschiedenen Adsorptionskapazität der Antigene an den Mikrotiterplatten bewirkt hätten werden können, wurden zusätzlich kompetitive Bindungsuntersuchungen durchgeführt. In einem ersten Test wurden Antikörper vorinkubiert mit verschiedenen Konzentrationen von ECA aus S. montevideo und LPS aus verschiedenen Arten (S. montevideo, E. coli 0111, S. minnesota und Serratia marcescens). Fig. 2 zeigt, daß nur lösliches ECA die Bindung des monoklonalen Antikörpers E21 an das festphasengebundene ECA wesentlich inhibierte. In einem zweiten Test wurde die Fähigkeit von ECA und verschiedenen LPS-Proben, die Bindung des Antikörpers E21 an E. coli LPS kompetitiv zu inhibieren getestet.

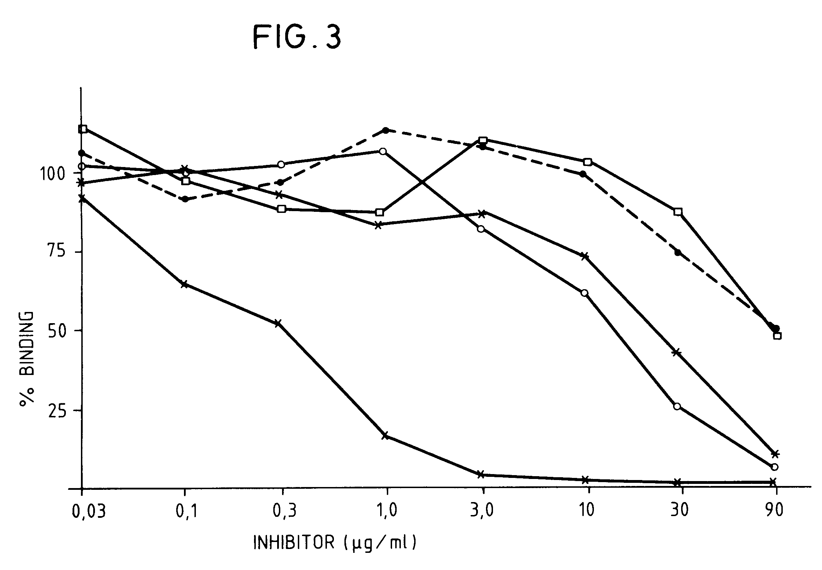

Fig. 3 zeigt, daß die Bindung von E21 an E. coli LPS durch sehr geringe ECA-Konzentrationen inhibiert wurde, wogegen LPS, das aus demselben Organismus isoliert worden war, nur eine 50%ige Hemmung der Bindung nur bei etwa 50fach höheren Konzentrationen zeigte. Heterologe LPS-Proben hatten sogar noch weniger Hemmwirkung. Diese Ergebnisse zeigen, daß die monoklonalen Antikörper ein Epitop erkennen, das bei ECA mehr hervortritt als bei den Lipopolysacchariden.

**Beispiel 6**

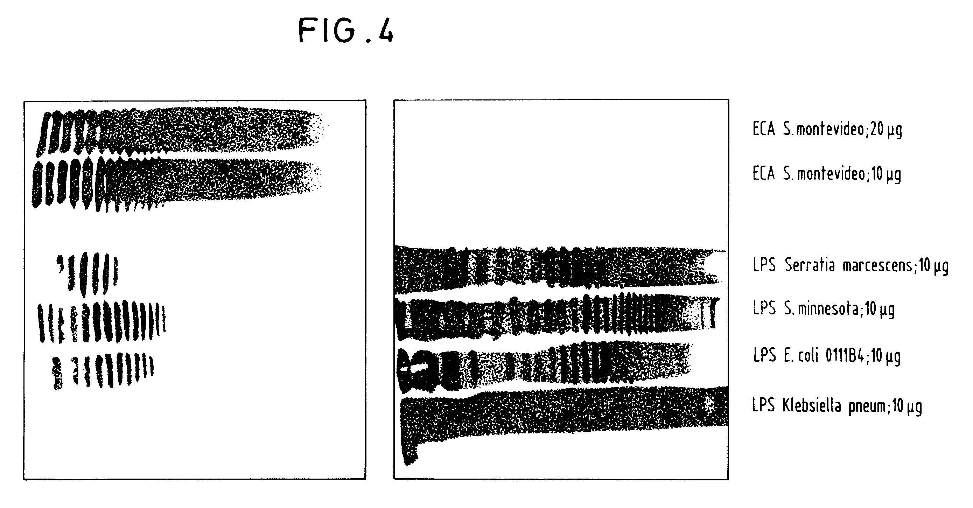

Gereinigtes ECA aus S. montevideo und LPS aus verschiedenen Arten (einschließlich S. montevideo) wurde einer SDS-Gelelektrophorese unterworfen und durch Western-Blotting und Silberfärbung sichtbar gemacht. Die Ergebnisse sind Fig. 4 zu entnehmen. Es wurde gefunden, daß LPS das typische leiterartige Muster nach der Anfärbung mit Silber ergab, wohingegen ECA nicht mit Silbernitrat gefärbt werden konnte. Das Sichtbarmachen der geblotteten Antigene mit dem Antikörper E21 zeigte ein kontinuierliches leiterartiges Muster von ECA-Molekülen, deren Molekulargewichtsbereich sich von sehr niedrigen bis zu hohen Molekulargewichten erstreckt. Im Gegensatz dazu reagierte der monoklonale Antikörper E21 mit LPS in Immunoblots nur mit wenigen Banden von hauptsächlich niedrigem Molekulargewicht. Die hohen Molekulargewichtsbanden von LPS reagierten überhaupt nicht, obwohl sie in ausreichenden Mengen vorhanden waren.

**Patentansprüche**

1. Verfahren zur Herstellung von gegen ECA (Enterobacterial Common Antigen) gerichteten monoklonalen Antikörpern,
   **dadurch gekennzeichnet,**
   daß man aus dem Blut von Spendern, bei denen eine Enterobacteriaceae-Infektion nachgewiesen wurde, B-Lymphozyten gewinnt, die Lymphozyten immortalisiert, die Zellen, die Antikörper mit der gewünschten Aktivität produzieren, selektioniert, züchtet und die Antikörper daraus gewinnt.

2. Verfahren nach Anspruch 1, **dadurch gekennzeichnet,** daß man zur Immortalisierung die Lymphozyten mit Myelomzellen humaner oder muriner Herkunft, mit subzellulären Vesikeln, die eine transformierende DNA enthalten, fusioniert oder mit Epstein-Barr-Virus transformiert.

3. Verfahren nach Anspruch 1 oder 2, **dadurch gekennzeichnet,** daß man zur Immortalisierung Lymphozyten mit Zellen der humanen Myelomzellinie SK0-007, ATCC CRL 8033 fusioniert.

4. Zellinie ECACC 89030311 (E21)

5. Zellinie ECACC 89030310 (L30)

6. Verwendung von gemäß einem der Verfahren von Anspruch 1 bis 3 oder aus den Zellinien ECACC 89030310 und/oder ECACC 89030311 gewonnenen monoklonalen Antikörpern zur Herstellung eines pharmazeutischen Präparats zur passiven Immunisierung gegen von Enterobacteriaceae hervorgerufenen Infektionen.

7. Verwendung von gemäß einem der Verfahren von Anspruch 1 bis 3 oder aus den Zellinien ECACC 89030310 und/oder ECACC 89030311 gewonnenen monoklonalen Antikörpern zum qualitativen oder quantitativen Nachweis von Enterobacteriaceae.

8. Arzneimittel zur Behandlung von von Enterobacteriaceae hervorgerufenen Infektionen, enthaltend gemäß dem Verfahren nach einem der Ansprüche 1 bis 3 erhaltene monoklonale Antikörper zusammen mit üblichen Träger- und Verdünnungsmitteln.

9. Arzneimittel nach Anspruch 8, enthaltend die aus den Zellinien ECACC 89030310 und/oder ECACC 89030311 gewonnenen monoklonalen Antikörper.

**Claims**

1. Process for the production of monoclonal antibodies directed against ECA (enterobacterial common antigen), characterised in that one obtains B-lymphocytes from the blood of donors in whom an Enterobacteriaceae infection has been detected, immortalises the lymphocytes, selections the cells with produce antibodies with the desired activity, cultures and obtains the antibodies therefrom.

2. Process according to claim 1, characterised in that for the immortalisation, one fuses the lymphocytes with myeloma cells of human or murine origin with subvesicles which contain a transforming DNA or transforms with Epstein-Barr virus.

3. Process according to claim 1 or 2, characterised in that, for the immortalisation, one fuses the lymphocytes with cells of the human myeloma cell line SKO-007, ATCC CRL 8033,

4. Cell line ECACC 89030311 (E21).

5. Cell line ECACC 89030310 (L30).

6. Use of monoclonal antibodies obtained according to one of the processes according to claim 1 to 3 or from the cell lines ECACC 89030310 and/or ECACC 890303011 for the production of a pharmaceutical preparation for the passive immunisation against infections brought about by Enterobacteriaceae.

7. Use of monoclonal antibodies obtained according to one of the processes according to claim 1 to 3 or from the cell lines ECACC 89030310 and/or ECACC 89030311 for the qualitative or quantitative detection of Enterobacteriaceae.

8. Medicaments for the treatment of infections brought about by Enterobacteriaceae, containing monoclonal antibodies obtained according to the process according to one of claims 1 to 3, together with usual carrier and dilution agents.

9. Medicaments according to claim 8, containing the monoclonal antibodies obtained from the cell lines ECACC 89030310 and/or ECACC 89030311.

**Revendications**

1. Procédé de production d'anticorps monoclonaux dirigés contre ECA (Enterobacterial Common Antigen), caractérisé en ce que l'on obtient des lymphocytes B à partir du sang de donneurs chez lesquels une infection à Enterobacteriaceae a été mise en évidence, on immortalise les lymphocytes, on sélectionne les cellules qui produisent des anticorps présentant l'activité souhaitée, on les cultive et on obtient les anticorps à partir de celles-ci.

2. Procédé selon la revendication 1, caractérisé en ce que, pour l'immortalisation, on fusionne les lymphocytes avec des cellules de myélome d'origine humaine ou murine, avec des vésicules subcellulaires qui contiennent un ADN transformant ou bien on les transforme avec le virus d'Epstein-Barr.

3. Procédé selon la revendication 1 ou 2, caractérisé en ce que, pour l'immortalisation, on fusionne les lymphocytes avec des cellules de la lignée cellulaire de myélome humaine SKO-007, ATCC CRL 8033.

4. Lignée cellulaire ECACC 89030311 (E21)

5. Lignée cellulaire ECACC 89030310 (L30)

6. Utilisation des anticorps monoclonaux obtenus par l'un des procédés selon les revendications 1 à 3 ou à partir des lignées cellulaires ECACC 89030310 et/ou ECACC 89030311 pour la production d'une préparation pharmaceutique pour l'immunisation passive contre des infections provoquées par des Enterobacteriaceae.

7. Utilisation d'anticorps monoclonaux obtenus par l'un des procédés selon les revendications 1 à 3 ou à partir des lignées cellulaires ECACC 89030310 et/ou ECACC 89030311 pour la mise en évidence qualitative ou quantitative d'Enterobacteriaceae.

8. Médicament pour le traitement d'infections provoquées par des Enterobacteriaceae, contenant des anticorps monoclonaux obtenus par le procédé selon l'une des revendications 1 à 3 ainsi que des véhicules et diluants courants.

9. Médicament selon la revendication 8, contenant les anticorps monoclonaux obtenus à partir des lignées cellulaires ECACC 89030310 et/ou ECACC 89030311.

# FIG. 1

FIG. 2

FIG. 3

# FIG.4

ECA S.montevideo;20 µg

ECA S.montevideo;10 µg

LPS Serratia marcescens;10 µg

LPS S.minnesota;10 µg

LPS E.coli 0111B4;10 µg

LPS Klebsiella pneum;10 µg